Europäisches Patentamt

European Patent Office

Office européen des brevets

(11) Publication number: **0 487 208 A1**

# EUROPEAN PATENT APPLICATION

(12)

(21) Application number: **91309741.6**

(22) Date of filing: **22.10.91**

(51) Int. Cl.⁵: **C07C 68/06**, C07C 69/96

(30) Priority: **22.11.90 GB 9025388**

(43) Date of publication of application:
**27.05.92 Bulletin 92/22**

(84) Designated Contracting States:
**AT BE CH DE DK ES FR GB GR IT LI LU NL SE**

(71) Applicant: **THE BRITISH PETROLEUM COMPANY P.L.C.**
**Britannic House, 1 Finsbury Circus**
**London EC2M 7BA(GB)**

(72) Inventor: **Ambler, Philip William**
**The British Petroleum Co. p.l.c., Chertsey Road**
**Sunbury-on-Thames, Middlesex, TW16 7LN(GB)**
Inventor: **Stewart, Nevin John**
**The British Petroleum Co. p.l.c., Chertsey Road**
**Sunbury-on-Thames, Middlesex, TW16 7LN(GB)**

(74) Representative: **Crack, Richard David et al**
**BP INTERNATIONAL LIMITED Patents Division Chertsey Road**
**Sunbury-on-Thames Middlesex TW16 7LN(GB)**

(54) **Production of carbonates.**

㊷ A process for the production of an alkyl aryl carbonate comprises reacting an alkanol with a diaryl carbonate at elevated temperature in the presence, as catalyst, of 1,5,7, triaza-bicyclo [4.4.0] dec-5-ene. The alkanol is preferably a $C_1$ to $C_8$ alcohol, more preferably one that is branched at the carbon atom carrying the hydroxyl group. The reaction is conveniently carried out in the liquid phase at 20 to 100°C using a molar excess of the alkanol. The process is particularly suitable for the reaction of t-butanol with diphenyl carbonate to form phenyl t-butyl carbonate.

EP 0 487 208 A1

The present invention relates to the production of alkyl aryl carbonates.

Alkyl aryl carbonates are potentially useful compounds. Thus US 4 600 408 proposes to use an alkyl phenyl carbonate as an anti-knock additive. It has been proposed, for example in Org. Prep. Proced. Int 1980, 12 (3-4), 242-3 to make mixed alkyl aryl carbonates by treatment of chloroformates with a corresponding alkanol and phenol. However chloroformates require phosgene, a toxic gas, for their synthesis.

It has been proposed in GB 2 038 321 to make carbonic acid esters without using phosgene by the reaction of an alkanol, a phenol and carbon monoxide. However although alkyl aryl carbonates can be produced in this way very substantial amounts of the dialkyl carbonate and the diaryl carbonate are produced in the same reaction.

As disclosed in JP 61-291545 diaryl carbonates can be synthesised from dimethyl carbonate, which itself can be made without the use of phosgene.

In the procedure known as transesterification a product ester is produced from a starting ester by reaction with an alkanol. It is, however, very difficult to transesterify diaryl carbonates to produce alkyl aryl carbonates, i.e. to replace only one of the aryl groups. Thus we have found that a variety of known transesterification catalysts give little or no reaction when attempts are made to produce alkyl aryl carbonates by transesterification of diaryl carbonates. An alternative procedure in which phenol is reacted with dialkyl carbonates in the presence of strong bases as mentioned in GB 1 499 530 but this is stated to give a low reaction rate and to produce high amounts of phenyl ethers as by-products.

There is therefore a need to find a method of producing alkyl aryl carbonates in good yields without large amounts of other products and without using phosgene gas.

EP 110 629 discloses the use of a cyclic amidine as a transesterification catalyst. Among the amidines mentioned is 1,5,7-triaza-bicyclo[4.4.0]dec-5-ene. The specification refers to this material as TBD. The use of amidines as catalysts for transesterification of both carboxylic acid esters and carbonic acid esters is mentioned. However while the use of aryl esters is mentioned in relation to the carboxylic acid esters, only alkyl and aralkyl esters of carbonic acid are disclosed, i.e. not compounds in which the aryl group is attached directly to the oxygen of the carbonate group. This is consistent with the known difficulty of transesterifying diaryl carbonates to form alkyl aryl carbonates.

We have now found that by selection of a specific catalyst it is possible to obtain alkyl aryl esters by transesterification of a diaryl carbonate with an alkanol.

According to the present invention the process for the production of an alkyl aryl carbonate comprises reacting an alkanol with a diaryl carbonate at elevated temperature in the presence of 1,5,7-triaza-bicyclo-[4.4.0]dec-5-ene as catalyst.

The alkanol is preferably a branched alkanol, preferably branched at the carbon atom carrying the hydroxy group because the product is more hindered and less likely to undergo a second displacement, i.e. higher selectivity to alkyl aryl carbonates is favoured. The branched alkanol may be a secondary alkanol but is preferably a tertiary alkanol.

The alkanol conveniently contains from 1 to 20 carbon atoms in the molecule, more preferably 1 to 8 carbon atoms in the molecule. Examples of alkanols which can be used are isopropanol and t-butanol. Methanol may also be used.

The diaryl carbonate may be of formula $R_1C_6H_4$-O-CO-O $C_6H_4R_2$ where $R_1$ and $R_2$ are the same or different and chosen from H, $C_1$ to $C_4$ alkyl, cycloalkyl, chlorine, $NO_2$ and other inert substituents. An example is di-p-Tolyl carbonate. It is preferred to use diphenyl carbonate.

The catalyst is 1,5,7-triaza-bicyclo[4,4,0]dec-5-ene.

The catalyst of the present invention and its preparation is described in EP 198680.

The reaction is conveniently carried out in the liquid phase. Where the reactants are not themselves liquid a solvent may be used for example dichloromethane.

The reaction may be carried out at a moderately wide range of temperatures for example from 20 ° to 100 °C, or preferably from 50 ° to 90 °C.

Preferably a molar excess of the alcohol with respect to the diaryl carbonate is employed, for example, a molar ratio of alcohol to carbonate of 2 to 20 is convenient, more conveniently 4-15.

The reactants may be combined before applying heat or in any order after reaction temperature is reached. Higher yields are obtained when the catalyst is added to the reaction mixture at about 70 to 80°C.

When the alcohol starting material is t-butanol the reaction has been found to be selective to the t-butyl aryl carbonate (ie, the second aryl group is not removed by t-butyl). When other alcohols such as methanol and isopropanol are used, the reaction can be controlled to obtain the alkyl aryl carbonate by adjustment of the molar excess of the alcohol and/or the temperature and/or the duration of the reaction. The invention will now be described with reference to the following Examples in which examples of the invention are identified by number and Comparative Tests, not according to the invention, are identified by letter.

Comparative Test A

Attempts were made to transesterify diphenyl carbonate with t-butanol using various basic catalysts which might be expected to have transesterification activity. The reactions were carried out as follows:

Diphenyl carbonate was mixed with t-butanol (10 fold molar excess) in a reactor fitted with a reflux condenser and heated at reflux temperature (80-86 ° C) in the presence of 5 mol % catalyst (calculated with respect to diphenyl carbonate). Reaction was monitored by GC analysis.

The results for catalysts are shown in Table 1.

TABLE 1

| Catalyst | Reaction Time | Percentage Yield |
|---|---|---|
| potassium t-butoxide | 6 hours | 0 |
| DBU | 3 hours | 7 |
| titanium tetra (isopropoxide) | 6 hours | 0 |
| 4-DMAP | 6 hours | L |
| TMG | 4 hours | L |
| pyridine | 4 hours | L |
| dipropylamine | 6 hours | 0 |
| imidazole | 4 hours | L |
| uncatalysed | 4 hours | 0 |

In the Table above L indicates that the yield was less than 1%. DBU is 1,8-diazabicyclo [5.4.0] undec-7-ene, 4-DMAP is 4-dimethyl aminopyridine, TMG is tetramethyl guanidine.

The percentage yield is the molar amount of phenyl t butyl carbonate produced divided by the theoretical amount of phenyl t butyl carbonate produced if all the diphenyl carbonate starting material were converted to phenyl t butyl carbonate x 100.

The above results indicate the great difficulty in carrying out base catalysed transesterification of diphenyl carbonate.

Example 1

This experiment again used a 10 fold molar excess of t-butanol and was carried out in the same way as in Comparative Test A except that the catalyst used was 1,5,7-triaza-bicyclo[4.4.0]dec-5-ene. The yield of phenyl t butyl carbonate by gas chromotography after 3 hours reaction time was 95% and the yield after 5 hours reaction time was 98%.

A comparison of the results for Comparative Test A with those for Example 1 shows the outstanding performance which can be obtained by the use of the catalyst of the present invention.

Example 2

Diphenyl carbonate (5g, 23.4 mmol) was introduced into a reaction vessel provided with a reflux condenser. Isopropanol (6 ml, 78.5 mmol) was then introduced, followed by 1,5,7-triaza-bicyclo[4.4.0]dec-5-ene (81 mg, 0.58 mmol). The reactants and the catalyst were mixed together and then heated to 60°C. The reaction vessel was allowed to cool after half an hour and the contents analysed by gas chromatography. This analysis indicated that over 50% of the diphenyl carbonate had reacted. The reaction product contained greater than 95% isopropyl phenyl carbonate.

Example 3

Diphenyl carbonate (5g, 23.4 mmol) was introduced into a reaction vessel followed by dichloromethane (3 ml), 1,5,7-triazabicyclo [4.4.0] dec-5-ene (81 mg, 0.58 mmol) and methanol (1.14 ml, 28.1 mmol). After mixing at 20 ° C for 40 minutes, GC analysis indicated that 30% of the carbonate had reacted with 83% selectivity to methyl phenyl carbonate.

Comparative Test B

An experiment was carried out as in Example 3 except that no catalyst was used. The conversion to methyl phenyl carbonate was less than 4% after 4.5 hours.

**Claims**

1. A process for the production of an alkyl aryl carbonate which process comprises reacting an alkanol with a diaryl carbonate at elevated temperature in the presence of 1,5,7-triaza-bicyclo[4.4.0]dec-5-ene as catalyst.

2. A process as claimed in Claim 1 wherein the diaryl carbonate is of formula $R_1C_6H_4$-O-CO-O $C_6H_4R_2$ where $R_1$ and $R_2$ are the same or different and are chosen from H, $C_1$ to $C_4$ alkyl, cycloalkyl, chlorine, $-NO_2$ and other inert substituents.

3. A process as claimed in Claim 1 or 2 wherein the alkanol contains from 1 to 8 carbon atoms.

4. A process as claimed in any one of the preceding claims wherein the alkanol is branched at the carbon atom carrying the hydroxyl group.

5. A process as claimed in Claim 4 wherein the alkanol is a tertiary alkanol.

6. A process as claimed in any one of the preceding claims wherein the process is carried out in the liquid phase at a temperature of from 20 to 100°C.

7. A process as claimed in Claim 6 wherein the process is carried out in the presence of a solvent for the reactants.

8. A process as claimed in any one of the preceding claims wherein the diaryl carbonate is diphenyl carbonate.

9. A process for the production of a phenyl alkyl carbonate selected from phenyl t-butyl carbonate, phenyl isopropyl carbonate and phenyl methyl carbonate which process comprises reacting diphenyl carbonate with an alkanol selected from t butyl alcohol, isopropanol and methanol as the case may be at elevated temperature in the presence of 1,5,7-triaza-bicyclo[4.4.0]dec-5-ene as catalyst.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 150 962 (BP CHEMICALS LTD)<br>* page 1, line 12 - page 3, line 25 *<br>* page 4, line 5 - line 17 *<br>--- | 1 | C07C68/06<br>C07C69/96 |
| D,A | EP-A-0 110 629 (BP CHEMICALS LTD)<br>* page 2, line 1 - page 3, line 15 *<br>--- | 1 | |
| D,A | GB-A-1 499 530 (SNAMPROGETTI SPA)<br><br>------ | 1 | |
| | | | **TECHNICAL FIELDS SEARCHED (Int. Cl.5 )**<br><br>C07C |

The present search report has been drawn up for all claims

| Place of search | Date of completion of the search | Examiner |
|---|---|---|
| THE HAGUE | 26 FEBRUARY 1992 | KINZINGER J.M. |

EPO FORM 1503 03.82 (P0401)